# EUROPEAN PATENT APPLICATION

(11) **EP 1 407 783 A1**
(43) Date of publication of application: **14.04.2004**
(21) Application number: 02741335.0
(22) Date of filing: 26.06.2002
(51) Int. Cl.: A61K 45/00, A61K 38/17, A61P 19/00, A61P 19/08, A61P 43/00

(54) **REMEDIES FOR HERNIA**

(30) Priority: 26.06.2001 JP 2001193239
(71) Applicant: Komori, Hiromichi, Koganei-shi, Tokyo 184-0015 (JP)
(72) Inventor: HARO, Hirotaka, Meguro-ku, Tokyo 152-0013 (JP)
(74) Representative: Luderschmidt, Schüler & Partner
(86) International application number: PCT/JP2002/006412
(87) International publication number: WO 2003/000287

(57) **Abstract**

There are provided herniated disc treatment agents developed as a result of molecular level analysis of the action mechanism of spontaneous resorption of herniated disc. Angiogenesis-inducing substances are administered to promote spontaneous resorption of hernia by inducing vascular tubule formation in herniated disc tissue.

## Description

### Technical Field

The present invention relates to hernia treatment agents comprising angiogenesis-inducing substances as active ingredients. More specifically, the invention relates to agents which treat intervertebral disc hernia by promoting spontaneous resorption of herniated disc when administered to the affected area of a herniated intervertebral disc, and to a treatment method for herniated disc using the agents.

### Background Art

The intervertebral discs, which are cartilage discs present between the vertebral bodies, each consist of an outer annulus fibrosus surrounding an inner nucleus pulposus, and are composed of cartilage components such as proteoglycans (including aggrecan), type II collagen and the like. An intervertebral disc loses elasticity by reduction in the water content of the disc as a result of aging for the most part, and this results in hardening and weakening of the annulus fibrosus and cartilaginous disc (Lipson S.J. & Muir H., Arthritis Rheum. 24(1981), p.12-21).
Degeneration of intervertebral discs occurs as a part of the normal changes of aging, and it has been reported that 80% of the intervertebral discs are degenerated by age 70 (Powell M.C. et al., Lancet 2(1986), p.1366-1367).

The major skeletal trouble associated with intervertebral disc degeneration is backache, which has been experienced by a very large proportion of the population. Intervertebral disc degeneration sometimes leads to protrusion of the intervertebral disc into the spinal canal, causing herniated disc. Herniated disc, and particularly lumbar herniated disc, is a common causative condition of lumbago and sciatic neuralgia, and according to recent statistics in Japan, the proportion of inpatient treatment cases for lumbar herniated disc is as high as 7.4 per 1000, with a wide age distribution from youth to the elderly. Nevertheless, many aspects of the mechanism of herniated disc remain unclear.

Magnetic resonance imaging (MRI) has been widely employed as a tool for diagnosis of herniated disc. Imaging of intervertebral disc tissue can be enhanced by using the contrast agent gadolinium-diethylenetriamine pentaacetic acid (Gd-DTPA), to allow more accurate diagnosis (Komori H. et al., Spine. 23(1998), p.67-73). The more accurate imaging of herniated disc tissue by MRI has permitted frequent detection of spontaneous resorption of herniated disc sections, i.e. intervertebral discs which have protruded into the spinal canal (Komori, H. et al., Spine. 21(1996), p.225-229). It has been reported that hernia masses show neovascularization and infiltration of inflammatory cells, primarily macrophages, in the cartilage matrix (Haro et al., Spine 21(1996), 647-652). However, the details regarding the mechanism of spontaneous resorption have not yet been elucidated.

### Problems to be Solved by the Invention

It is an object of the present invention to analyze the action mechanism for spontaneous resorption of herniated disc on the molecular level and discover a substance which promotes it, in order to provide intervertebral disc hernia treatment agents and a treatment method.

### Means for Solving the Problems

As a result of diligent research directed toward achieving the object stated above, the present inventors have completed the present invention upon discovering that vascular tubules are formed in herniated disc tissue although adult intervertebral discs lack a vasculature, that numerous macrophages aggregate in the angiogenic herniated disc tissue prior to spontaneous resorption, and that the vascular tubule formation can be brought about in intervertebral disc tissue by substances which induce angiogenesis.

Specifically, upon examining in detail the expression of substances in intervertebral disc tissues and macrophages on a molecular level, it was found that substances which induce angiogenesis are key substances in spontaneous resorption of herniated disc.

The present invention therefore relates to herniated disc treatment agents comprising angiogenesis-inducing substances as active ingredients. According to the invention, any drug agents known to induce angiogenesis may be used as active ingredients for the hernia treatment agents of the invention. As examples of angiogenesis-inducing substances there may be mentioned VEGF (vascular endothelial cell growth factor), aFGF (acidic fibroblast growth factor), bFBF (basic fibroblast growth factor), PDGF (platelet-derived growth factor), EDF (epidermal growth factor), TGF-β (transforming growth factor-β), TNF-α (tumor necrosis factor-α) and IL-1β (interleukin-1β). Preferred angiogenesis-inducing substances are VEGF, bFGF and PDGF, with VEGF being more preferred.

MMP-3, also known as stromelysin-1 and believed to be involved in spontaneous resorption of herniated disc, is a potent proteoglycanase. Its proteolytic enzyme action breaks down the cartilaginous extracellular matrix and leads to herniated disc resorption. Thus, a more preferred herniated disc treatment comprises administration of a protease after promoting angiogenesis with an angiogenesis-inducing substance. One mode of the present invention, therefore, is a treatment agent which combines an angiogenesis-inducing substance with a protease. The protease may be any of the publicly known proteases used in the field of medicine. As examples of such proteases there may be mentioned collagenase, gelatinase, matrix metalloproteases (for example, MMP-3, MMP-7, MMP-13 (collagenase-3), MT1-MMP (MMP-14) and the like), papain, trypsin, chymotrypsin, plasmin and calpain. Matrix metalloproteases (extracellular matrix proteases) are preferred, with MMP-3 and MMP-7 being particularly preferred.

According to a preferred mode, the invention relates to herniated disc treatment agents and a treatment method comprising a combination of one angiogenesis-inducing substance selected from the group consisting of VEGF and bFGF with one protease selected from the group consisting of MMP-3, MMP-7 and MMP-13.

The herniated disc treatment agent of the invention is administered parenterally. For intervertebral puncture, for example, a puncture needle is percutaneously inserted into the intervertebral disc under radiofluoroscopy. An inner tube is then inserted in the direction of the protruded herniated disc for selective puncture of the protruded hernia, and the drug of the invention is injected. It may also be administered by injection into the epidural space. The herniated disc treatment agent of the invention may be administered in this manner to induce angiogenesis in herniated disc tissue and thereby promote spontaneous resorption of the herniated disc.

The protease may be administered together with, prior to or after the angiogenesis-inducing substance. Administration upon inducement of angiogenesis is a superior treatment method. The protease may be directly administered to the herniated disc site, or it may be administered systemically. In the case of systemic administration, any of various conventional methods of administration may be used, such as oral, subcutaneous, intramuscular, intravenous or intraabdominal administration. Methods for simultaneous administration of the angiogenesis-inducing substance and the protease include (a) a method of directly administering the angiogenesis-inducing substance and the protease to the herniated disc site, and (b) a method of administering the angiogenesis-inducing substance to the affected site and administering the protease systemically. Methods for administration of the angiogenesis-inducing substance and protease at different times include (a) a method of direct administration of both, and (b) a method of administering the angiogenesis-inducing substance to the affected site and administering the protease systemically.

When using a herniated disc treatment agent of the invention, it is preferred to employ magnetic resonance imaging (MRI) using a contrast agent, and preferably a contrast agent which can enhance imaging of cartilage tissue or fluids (for example, Gd-DTPA), for accurate diagnosis of the condition and site of nerve compression in the patient, and the severity of the herniated disc.

Another mode of the invention, therefore, is a herniated disc treatment method which comprises:
(a) performing MRI on a potential herniated disc patient,
(b) in the event that an herniated disc is found in the patient, administering to the herniated disc site an angiogenesis-inducing substance of the invention which is suitable for the condition, and
(c) if necessary administering a protease, to promote spontaneous resorption of the hernia tissue.

The dosage of a herniated disc treatment agent of the invention will differ depending on the severity of the hernia, the method of administration, the condition of the patient and the type of angiogenesis-inducing substance. The angiogenesis-inducing substance is administered directly to the affected site, usually at a per dose range of about 1 µg to 100 mg. The number of doses is preferably from one to several doses, but more may be administered depending on the extent of angiogenesis. When the protease is directly administered to the affected site, it may be administered at a per dose range of about 1 µg to 100 mg, and when administered systemically, in a per dose range of about 1-3,000 mg. The number of doses may be determined according to the need. The administration of the angiogenesis-inducing substance may also be followed by administration of the protease alone for several days to several months. The daily dosage and number of doses may be determined as appropriate for each case.

The present invention will now be explained in greater detail through the following examples, with the understanding that these examples are in no way limitative on the invention.

### Examples

### Example 1 Immunohistological observation of intervertebral disc hernia

The hernia tissue samples used were extracted from lumbar herniated disc patients (10: 7 male and 3 female; average age: 32.0 years [range 21-51 years]) who had undergone surgical treatment. The extracted hernia tissues were fixed in 4% paraformaldehyde at 4°C for 3 hours and then embedded in paraffin. Preparations of the samples were made and washed in phosphate-buffered saline containing 0.03% saponin (S-PBS), and were then treated with 0.6% hydrogen peroxide in methanol to remove the endogenous peroxidase activity. They were then incubated with anti-factor VIII antibody, anti-CD14 antibody, anti-human VEGF antibody, anti-human VEGF receptor-1 (Flt-1) antibody or anti-human VEGF receptor-2 (KDR-flk-1) antibody (R&D System, Minneapolis, MN). The antibodies were all murine, and mouse IgG (Sigma, St. Louis, MI) was used as a negative control. The sections were then washed with S-PBS, incubated with biotinylated anti-mouse IgG antibody (Histofine Immunohistological Staining Kit, Nichirei Co.) for 10 minutes, and then incubated for 5 minutes with addition of peroxidase-labeled streptavidin prior to color development.

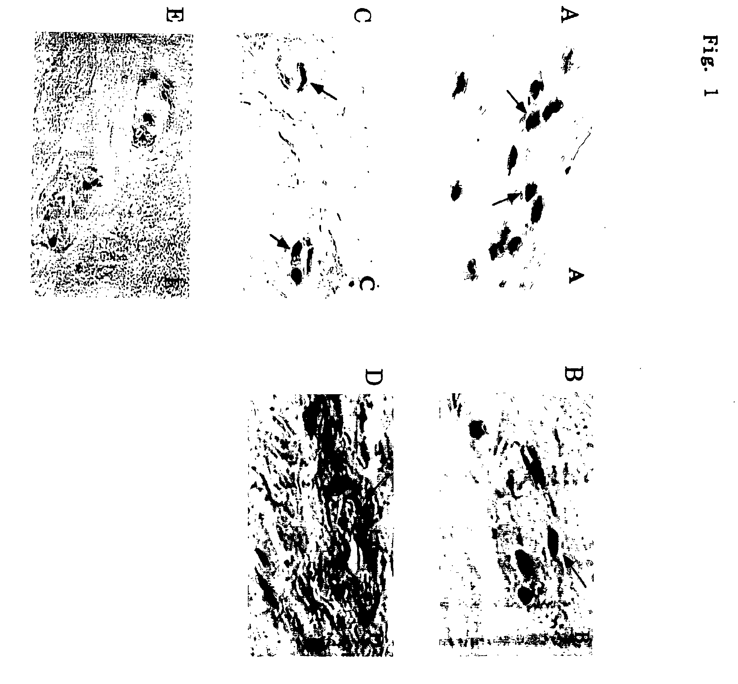

As a result, the hernia tissue was found to contain abundant vascular endothelial cells, which were positive for the endothelial cell-specific marker factor VIII, while infiltration of numerous CD-14-positive macrophages was also seen in the herniated disc tissue. VEGF, an endothelial cell-specific mitogen and an important angiogenesis factor, was observed in all of the samples and its expression was detected in both the macrophages and intervertebral disc cells (Figs. 1A and B). Expression of VEGFR-1 and VEGFR-2 was detected on the vascular endothelial cells of seven of the samples (Figs. 1C and D). No coloration was found in any of the samples with the negative control IgG (Fig. 1E). A large number of cells positive for VEGF, VEGFR-1 and VEGFR-2 were observed in hernia tissues that had protruded into the epidural space of the vertebral canal. This indicated that angiogenesis-inducing substances such as VEGF play an important role in angiogenesis in hernia tissue.

### Example 2 Expression of angiogenesis-inducing substances in intervertebral disk hernia model

As interaction between intervertebral disc tissue and macrophages was demonstrated by Example 1, the two were then cocultured together. The results of this coculturing system approximated those with human herniated disc, indicating its suitability as an *in vitro* model for acute stage herniated disc.

### 1) Macrophages and intervertebral disc tissue

Mice were intraabdominally injected with 2 ml of PBS containing 3% thioglycolate medium (Difco, Detroit, MI, USA). After four days, the activated macrophages were harvested by peritoneal lavage. Intervertebral disc tissue was sampled from the murine coccyx.

### 2) Coculturing

### [1] Direct culturing system

The sampled intervertebral disc tissues (5 discs/35 mm dish) or peritoneal macrophages (1 x 106/ml) were incubated alone or together in 5 ml of OPTI-MEM medium containing 50 mg/ml gentamycin and 0.25 µg/ml Fungizone (Gibco BRL, Grand Iand, New York, USA) for 2 days with 5% CO₂ at 37°C.

### [2] Transwell culturing system

Cell culture inserts (8.0 µm, Falcon, Franklin Lakes, NJ) were placed in wells of a 6-well plate (Corning Costar, Cambridge, MA) to form transwell chambers. The disc tissues were then transferred to the cell culture inserts, and the macrophages were transferred to the bottom of each well. After adding 5 ml of OPTI-MEM medium containing 50 mg/ml gentamycin and 0.25 µg/ml Fungizone to each well, the intervertebral disc tissues and macrophages were cocultured for 4 days at 37°C with 5% CO₂. In order to confirm the effect of TNF-α, a coculturing experiment was also conducted with addition of anti-TNF-α neutralizing antibody to the medium.

### 3) Analysis by Western blotting

Each medium was collected and the total protein was quantitated using a protein assay kit (Bio-Rad, Hercules, CA). The medium samples (32 µg protein) were subjected to SDS-PAGE (10% acrylamide gel) under reducing conditions, and the protein was transferred onto a nitrocellulose membrane (Nitro ME, MSI, Westborough, MA) or PVDF membrane (Bio-RAD). The membranes were blocked with 5% skim milk powder in TBS containing 0.05% Tween 20 (TBS-T), and then washed with TBS-T. Next, the membranes were incubated for one hour with biotinylated anti-mouse IgG, and then washed and incubated for one hour with peroxidase-conjugated streptavidin (Jackson Immuno-research, West Grove, PA). The immunoreactive proteins were identified by chemiluminescence using an ECL kit (Amersham Pharmacia Biotech, Buckinghamshire, UK). Recombinant expressed VEGF was used as a positive control.

### 4) Analysis by RT-PCR

VEGF expression was detected by RT-PCR. RT-PCR for GAPDH was conducted simultaneously as a control. The following primers were used for the PCR:

[VEGF]
Forward primer: 5'-GACAAGCTTATGAACTTTCTGCTCTCTTGGG-3' (SEQ
ID NO:1)
Reverse primer: 5'-GACTCTAGATCACCGCCTTGGCTTGTCACAT-3' (SEQ
ID NO:2)

[GAPDH]
Forward primer: 5'-AATGGTGAAGGTCGGTGTGA-3' (SEQ ID NO:3)
Reverse primer: 5'-TGGTGAAGACACCAGTAGAC-3' (SEQ ID NO:4)

The presence of VEGF or GAPDH mRNA yields fragments of 492 bp and 564 bp, respectively.

Total RNA was extracted from intervertebral disc tissues or macrophages, cultured alone or cocultured in the transwell culturing system described in [2] above, using a modified guanidine isothiocyanate procedure. One µg of the total RNA was incubated at 65°C for 10 minutes with oligo d(T) primers (Gibco BRL) and then immediately chilled on ice. A cDNA synthesis reaction buffer (final concentrations: 50 mM Tris-HCl, 75 mM KCl, 3 mM MgCl₂, 10 mM DTT), DL-dithiothreitol (DTT, Promega), ribonuclease inhibitor (Promega), dNTPs (Promega) and MMLV-reverse transcriptase (Promega) were added and the reaction mixture was incubated at 37°C for 1 hour, after which the cDNA synthesis reaction was suspended by boiling for 2 minutes and then chilling on ice. 5 µl of the cDNA product was supplied for PCR. The cDNA mixture was supplemented with 10 pmol of 5' and 3' primers, 1OX reaction buffer (50 mM Tris-HCl, 100 mM NaCl, 0.1 mM EDTA, 1 mM DTT, 50% glycerol and 1.0% Triton X-100, Promega), MgCl₂ (1.5 mM), dNTPs (0.25 mM) and 1.5 U of Ex Taq polymerase (Takara). The PCR reaction was conducted for 30 cycles, with each cycle consisting of 94°C for 30 seconds, 60°C for 30 seconds and 72°C for 1 minute. After the PCR, 10 µl of the reaction mixture was electrophoresed on 2.0% agarose gel (1X TBE) and the products were visualized by ethidium bromide staining.

### 5) Results

### [1] Western blotting

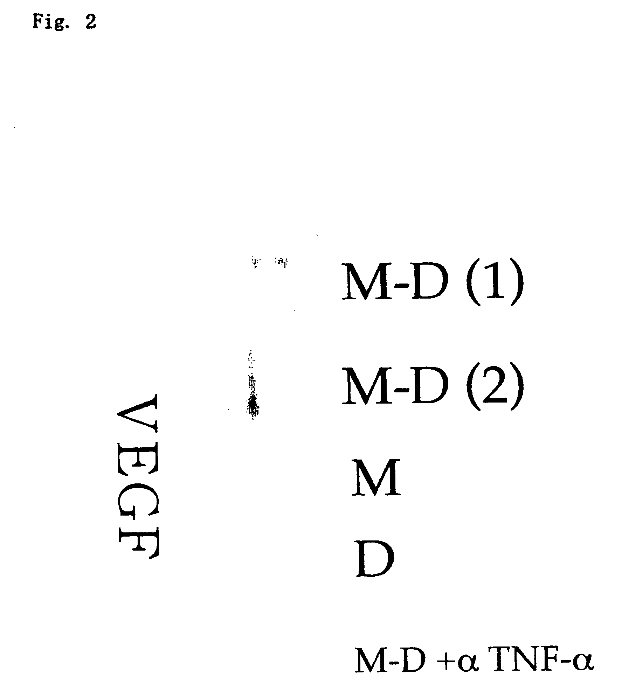

The intervertebral disc tissues or macrophages cultured alone produced very low levels of VEGF, but coculturing of the intervertebral disc tissues and macrophages resulted in high levels of VEGF production in both the direct coculturing system and transwell culturing system (Fig. 2). Also, addition of anti-TNF-α neutralizing antibody to the medium strongly inhibited the effect of coculturing, indicating that TNF-α is involved in the interaction between macrophages and intervertebral disc tissue (Fig. 2).

### [2] RT-PCR

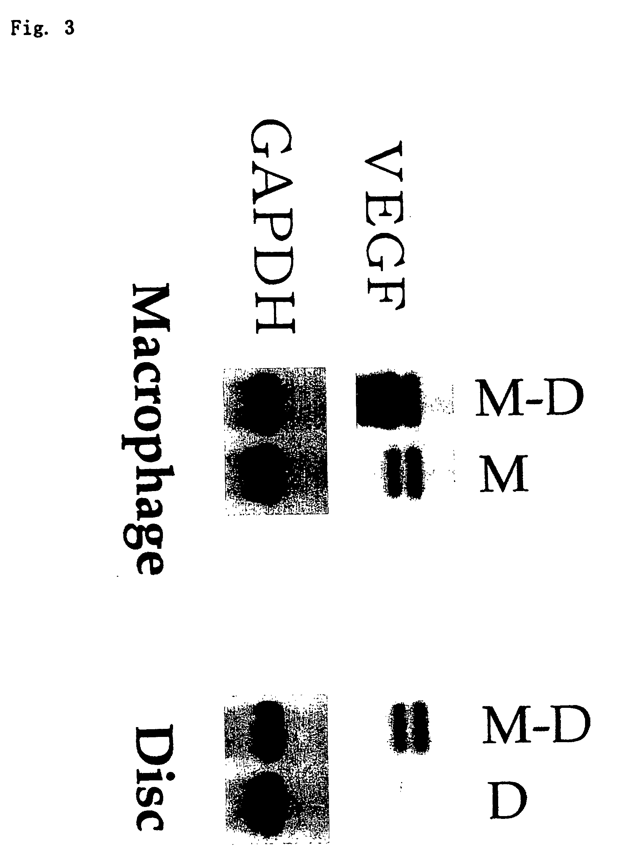

Although VEGF was also expressed in the macrophages cultured alone, higher levels of expression were found under coculturing with the intervertebral disc tissues (Fig. 3). Expression was also found with culturing of the intervertebral disc tissues alone, but coculturing with macrophages produced a notable increase in VEGF expression (Fig. 3). These results are consistent with those of the Western blotting analysis.

### Example 3 In vitro angiogenesis assay

An *in vitro* angiogenesis assay indicative of neovascularization has recently been developed for screening of substances which stimulate or inhibit angiogenesis (Bishop E.T. et al., Angiogenesis 3(2000), p.335-344). This assay was used to test the angiogenic activity of the media in which the intervertebral disc tissues and macrophages had been cultured.

Cultures of endothelial cells and dermal fibroblasts at the earliest stages of tubule formation were seeded in a 24-well plate, and then 1:1 mixtures of fresh medium and each of the media obtained in Example 2 were added for incubation for 11 days, upon which the degree of angiogenesis was observed. Recombinant VEGF and Suramin (TCS Biologicals) were used as positive and negative controls, respectively. Upon completion of the incubation period, the cells were fixed in 70% ethanol at room temperature and the formed tubules were stained with anti-CD31 (PECAM-1) antibody and BCIP/NBT.

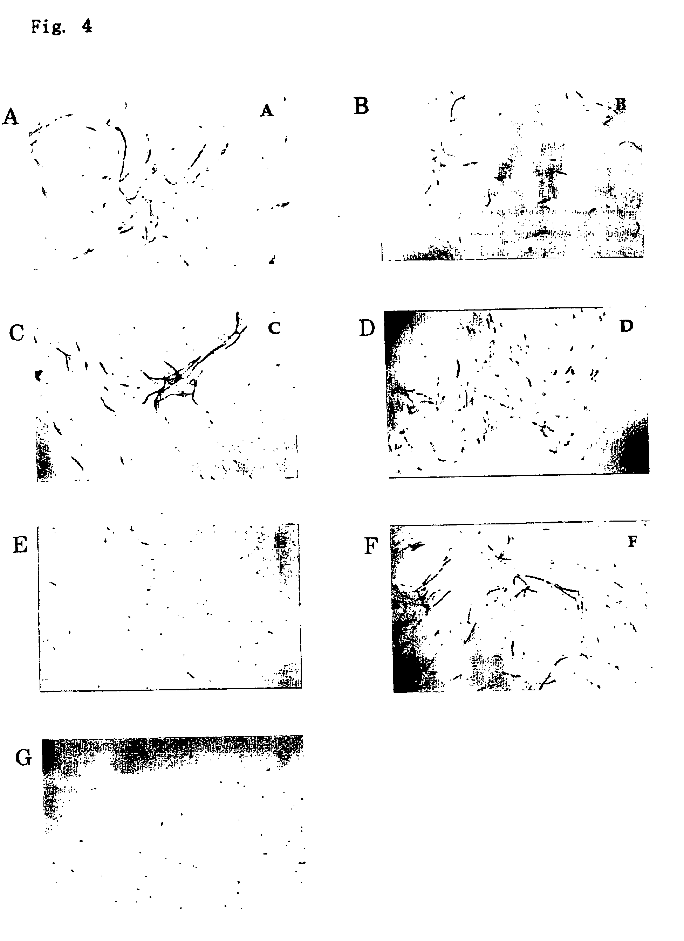

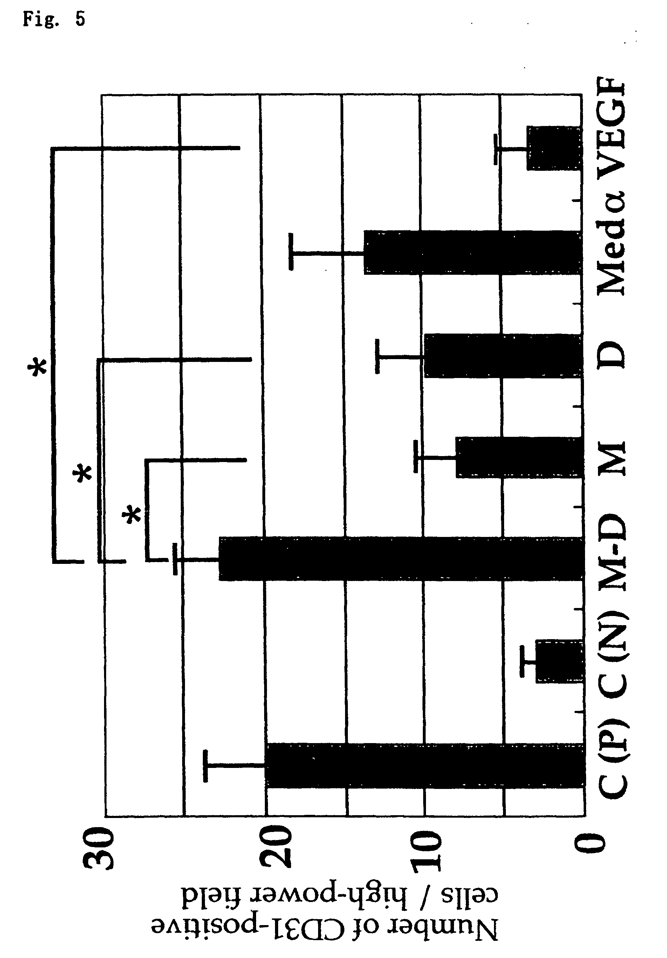

As a result, tubule formation was observed in the medium alone and in the media of the singly cultured intervertebral disc tissues or macrophages (Figs. 4-A, B, C). However, the media derived from the cocultured intervertebral disc tissues and macrophages exhibited abundant tubule network formation (Fig. 4-D). Similar results were obtained with addition of the recombinant VEGF used as positive control (Fig. 4-F), but virtually no tubule formation was found with the Suramin negative control (Fig. 4-E). Treatment of the cocultured media with anti-VEGF neutralizing antibody produced inhibition of the angiogenesis-inducing effect (Fig. 4-G). Furthermore, the macrophage and CD31-positive cell counts indicated a 2.9-fold increase in macrophages and a 2.3-fold increase in CD31-positive cells in the coculture media compared to the single culture media (Fig. 5). These results suggest that angiogenesis is significantly accelerated by coculturing of intervertebral disc tissue and macrophages.

### Example 4 Expression of angiogenesis-inducing factors in intervertebral disc hernia model

The intervertebral disc tissues and macrophages were cocultured in the same manner as Example 2, and expression of TNF-α, VEGF, MMP-7, MMP-3 and, as a control, GAPDH, was detected by RT-PCR.

The VEGF and GAPDH primers used for the PCR reaction were the same as in Example 2. The following TNF-α, MMP-3 and MMP-7 primers were used.

[TNF-α]
Forward primer: 5'-CAGACTCTTCCCTGAGGTGC-3' (SEQ ID NO:5)
Reverse primer: 5'-TCTCTCAATGACCGTAGGGC-3' (SEQ ID NO:6)

[MMP-3]
Forward primer: 5'-TTCTCCAGGATCTCTGAAGGAGAGG-3' (SEQ ID
NO:7)
Reverse primer: 5'-ATTTGGTGGGTACCACGAGGACATC-3' (SEQ ID
NO:8)

[MMP-7]
Forward primer: 5'-TGGAGTGCCAGATGTTGCAG-3' (SEQ ID NO:9)
Reverse primer: 5'-TTTCCATATAGCTTCTGAATGCCT-3' (SEQ ID
NO:10)

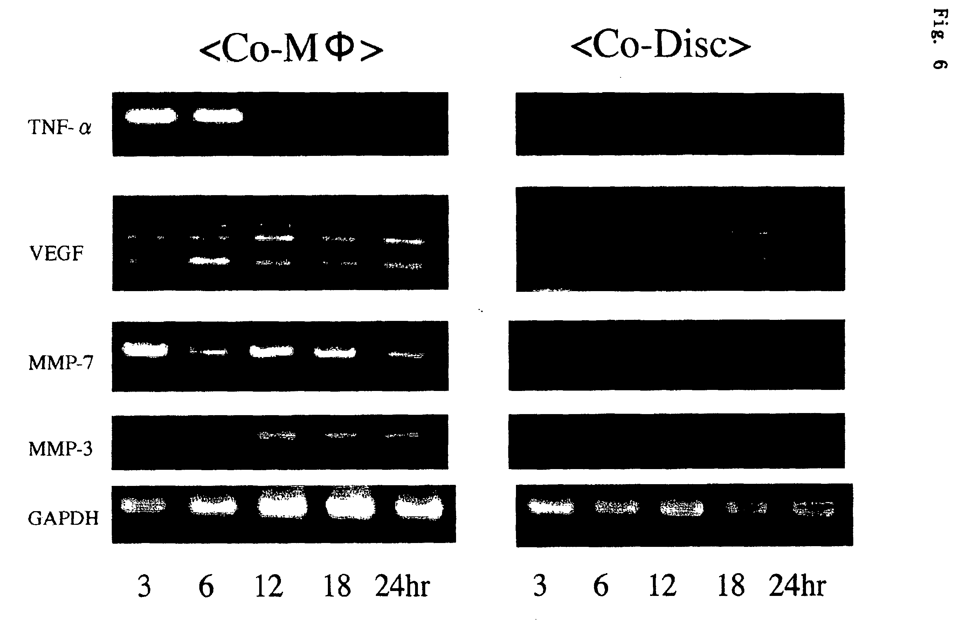

The results are shown in Fig. 6. In the cocultured macrophages, expression of TNF-α, VEGF and MMP-7 was confirmed after 3 hours while expression of MMP-3 was confirmed after 12 hours. In the cocultured intervertebral disc tissues, expression of VEGF and MMP-3 was confirmed after 3 hours while expression of TNF-α and MMP-7 was confirmed after 6 hours.

### Example 5 Confirmation of MMP-3 expression regulation by VEGF

### (1) Object

VEGF-neutralizing antibodies were used to confirm whether expression of MMP-3 and MMP-7 in hernia tissue is regulated by VEGF.

### (2) Harvesting of mouse macrophages

Mice were intraabdominally injected with 2 ml of PBS containing 3% thioglycolate, the peritoneal fluid was removed after 4 days, and the activated macrophages were harvested after centrifugation.

### (3) Organ culture of intervertebral disc

Intervertebral disc tissues were obtained from murine coccyges under a microscope after removing the soft tissue. Cell culture inserts (8.0 mm, Falcon, Franklin Lakes, NJ) were placed in the wells of 6-well culturing dishes and the coccygeal intervertebral discs were cocultured in the inserts with macrophages (1 x 106/ml) placed at the bottom of each culture dish. Anti-VEGF antibody (1 ng/mL) was administered every 6 hours after the start of culturing, total RNA was extracted from the intervertebral discs and macrophages every 3, 6, 12, 18 and 24 hours after the start of culturing, and expression of VEGF, MMP-3 and MMP-7 was examined by RT-PCR. Separately, anti-VEGF antibody (1 ng/mL) was added at the start of culturing, and then after 12 hours, recombinant VEGF (1 ng/mL) was added and expression of VEGF, MMP-3 and MMP-7 in the intervertebral discs and macrophages was periodically examined.

### (4) Results

While expression of VEGF on the mRNA level was detected with addition of VEGF neutralizing antibody, expression of MMP-3, which had been augmented after 12 hours from the start of culturing, was inhibited. However, addition of recombinant VEGF to the VEGF neutralizing antibody-added cultures completely eliminated this inhibition on MMP-3 expression. This demonstrated an important relationship between MMP-3 expression and VEGF.

### Effect of the Invention

According to the present invention, it has been demonstrated that vascular tubule formation in herniated disc tissue plays a crucial role in spontaneous resorption of herniated disc, and that vascular tubule formation in the tissues is significantly promoted by substances capable of inducing vascularization, i.e. by substances with angiogenesis-stimulating activity. Hernia treatment agents according to the invention which comprise such substances as active ingredients promote spontaneous resorption of intervertebral disc hernia and are therefore highly useful for herniated disc therapy.

### Brief Description of the Drawings

Fig. 1 is micrograph showing the results of immunohistological analysis of human intervertebral disc hernia tissue, which indicates staining of hernia tissue with anti-human VEGF antibody (A, B), anti-VEGFR-1 antibody (C) and anti-VEGFR-2 antibody (D), and lack of staining with the control IgG.
Fig. 2 shows the results of Western blot analysis of VEGF protein expression in intervertebral disc tissue/macrophage coculture, wherein VEGF expression was increased by coculturing (M-D(1), M-D(2)) but not by single culturing, thus indicating that VEGF expression is inhibited by anti-TNF-α antibody treatment.
Fig. 3 shows the results of RT-PCR analysis of VEGF mRNA expression by coculturing of intervertebral disc tissue and macrophages, indicating increased expression with the cocultured macrophages and induced expression in the intervertebral disc tissue.
Fig. 4 is a micrograph showing the results of the *in vitro* angiogenesis assay, indicating that angiogenesis was strongly stimulated in the cocultured media (D) and with recombinant VEGF (F), and that this effect was inhibited by anti-VEGF neutralizing antibody.

Fig. 5 is a graph showing the number of CD31-positive cells upon incubation with control medium alone (Med) or using medium from macrophages (M) or intervertebral disc tissues (D) grown alone or in coculture (M-D), as an *in vitro* angiogenesis assay. Anti-VEGF antibody was added to the coculture medium (αVEGF), recombinant VEGF (10 ng/ml) was used as a positive control (C(P)) and Suramin (100 mM) was used as a negative control (C(N)). Data were analyzed using the Mann-Whitney test and are shown as mean ±SD. Significant difference from coculture (M-D): P<0.01.

Fig. 6 shows the results of RT-PCR analysis of mRNA expression for TNF-α, VEGF, MMP-7, MMP-3 and, as a control, GAPDH, with cocultures of intervertebral disc tissues and macrophages, in an alignment allowing comparison based on the time after start of culturing. <Co-MΦ> represents the cocultured macrophages, and <Co-Disc> represents the cocultured intervertebral disc tissues.

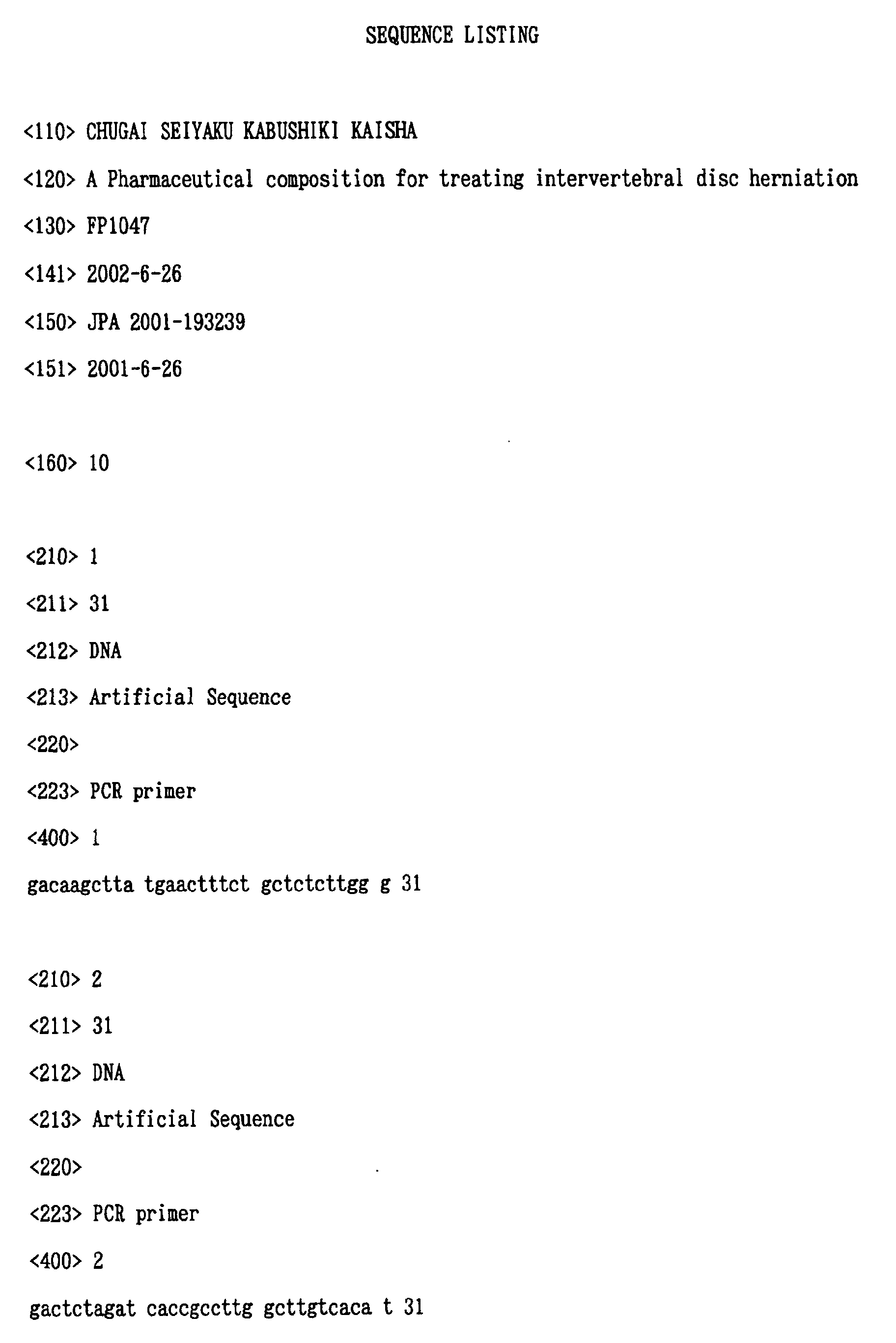

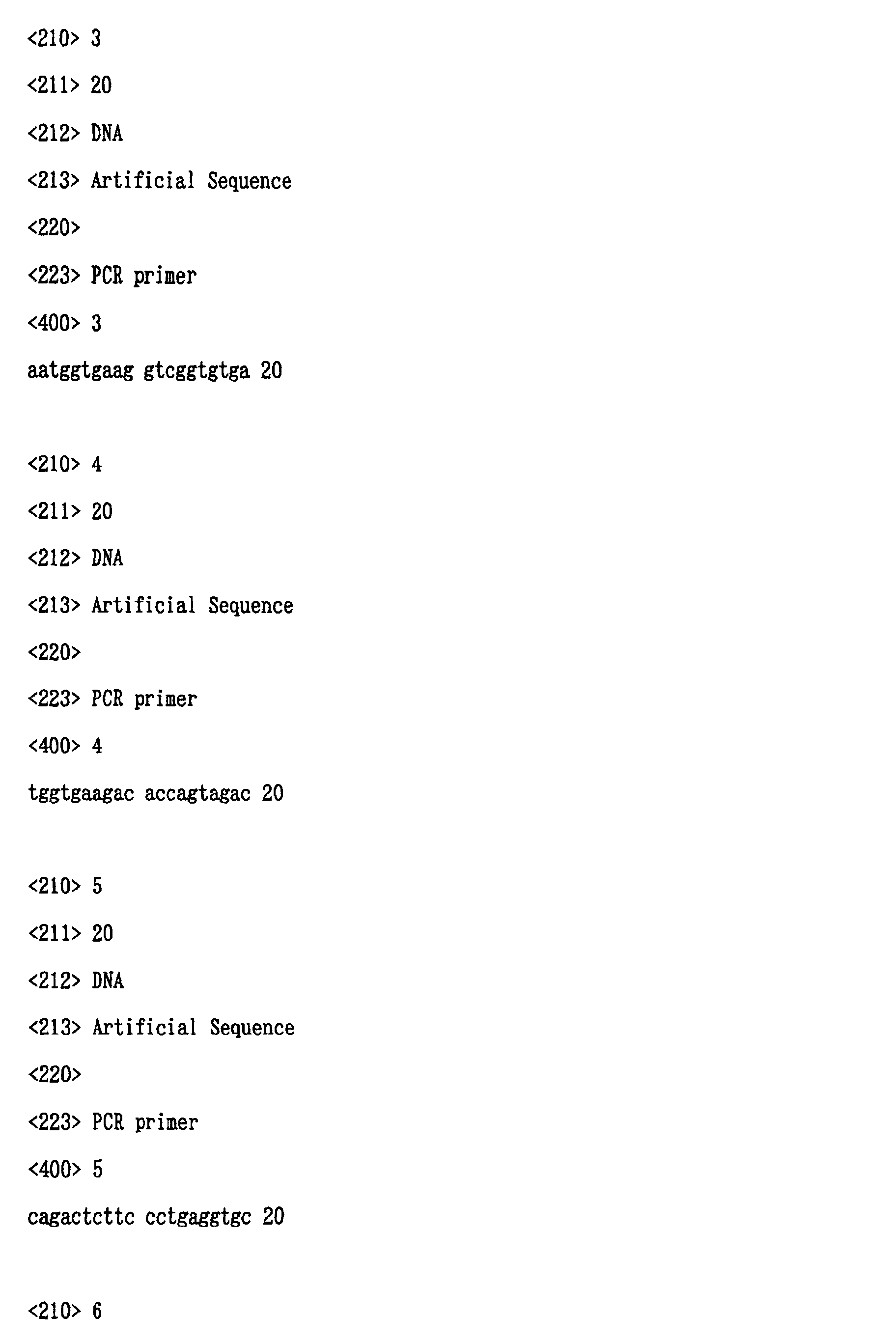

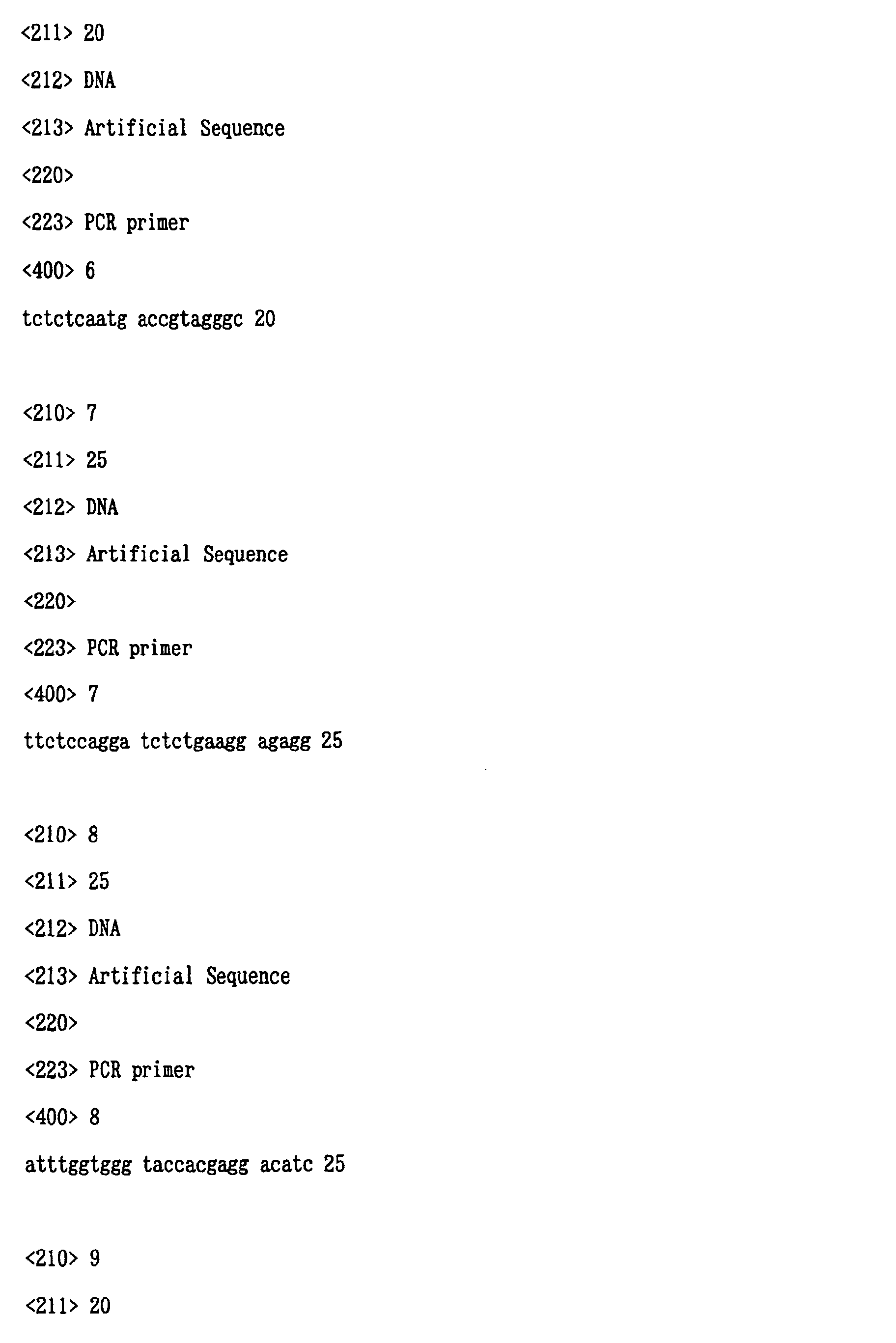

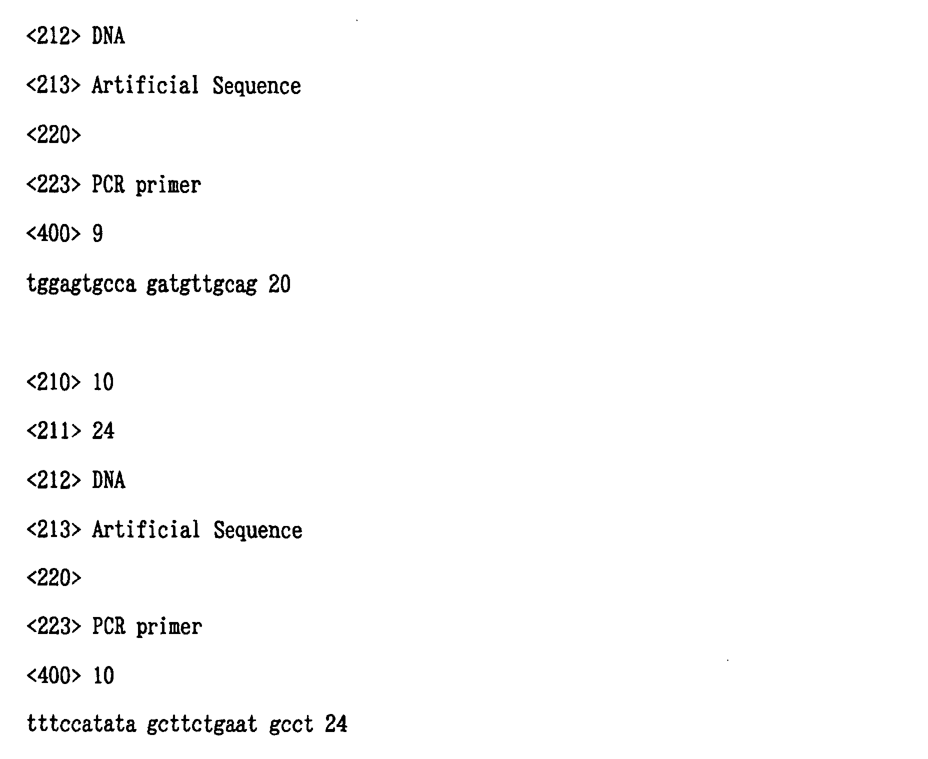

## Claims

1. A hernia treatment agent comprising an angiogenesis-inducing substance as an active ingredient.

2. A hernia treatment agent according to claim 1, wherein the angiogenesis-inducing substance is selected from the group consisting of VEGF, aFGF, bFGF, PDGF, EGF, TGF-β, TNF-α and IL-1β.

3. A hernia treatment agent according to claim 2, wherein the angiogenesis-inducing substance is VEGF.

4. A hernia treatment agent according to any one of claims 1 to 3, which is used in combination with a protease.

5. A hernia treatment agent according to claim 4, wherein the protease is an extracellular matrix protease.

6. A hernia treatment agent according to claim 5, wherein the active ingredient is a combination of one angiogenesis-inducing substance selected from the group consisting of VEGF and bFGF, and one protease selected from the group consisting of MMP-3, MMP-7 and MMP-13.

7. A hernia treatment agent according to any one of claims 1 to 6, which is directly administered to a hernia site.

8. A hernia treatment method which comprises administering an angiogenesis-inducing substance and a protease.

9. A hernia treatment method according to claim 8, wherein the angiogenesis-inducing substance and the protease are administered simultaneously.

10. A hernia treatment method according to claim 9, wherein the angiogenesis-inducing substance is directly administered to the affected site, and the protease is administered systemically.

11. A hernia treatment method according to claim 8, wherein the angiogenesis-inducing substance and the protease are administered at different times.

12. A hernia treatment method according to claim 11, wherein the angiogenesis-inducing substance is directly administered to the affected site, and the protease is administered systemically.
